# EUROPÄISCHE PATENTSCHRIFT

(11) **EP 1 418 874 B1**
(45) Veröffentlichungstag und Bekanntmachung des Hinweises auf die Patenterteilung: **04.01.2006**
(21) Anmeldenummer: 02747422.0
(22) Anmeldetag: 24.06.2002
(51) Int. Cl.: A61F 13/62

(54) **BEFESTIGUNGSELEMENT FÜR HYGIENEARTIKEL UND ENDLOSBAHN FÜR SEINE HERSTELLUNG**
FASTENING ELEMENT FOR HYGIENE ARTICLES AND ENDLESS TAPE FOR THE PRODUCTION OF SAID ELEMENT
ELEMENT DE FIXATION POUR ARTICLES D'HYGIENE ET BANDE SANS FIN SERVANT A LA FABRICATION DUDIT ELEMENT

(30) Priorität: 18.08.2001 DE 10140621
(43) Veröffentlichungstag der Anmeldung: 19.05.2004
(73) Patentinhaber: Paul Hartmann Aktiengesellschaft, 89522 Heidenheim (DE)
(72) Erfinder: WENDELSTORF, Carsten, 56068 Koblenz (DE); MANGOLD, Rainer, 89542 Herbrechtingen (DE)
(74) Vertreter: Friz, Oliver
(86) Internationale Anmeldenummer: PCT/EP2002/006945
(87) Internationale Veröffentlichungsnummer: WO 2003/015683

(56) Entgegenhaltungen:
- EP-A- 1 151 736
- WO-A-98/22069
- DE-U- 20 005 920
- FR-A- 2 267 058
- GB-A- 1 441 567
- US-A- 4 670 012
- US-A- 5 234 517
- US-A- 6 030 373

## Beschreibung

Die Erfindung betrifft ein Befestigungselement für Hygieneartikel, insbesondere für Windeln oder für Inkontinenzartikel, zum lösbaren Schließen im an einen Benutzer angelegten Zustand, wobei das Befestigungselement eine erste Längsrichtung und eine zweite Querrichtung aufweist und in Form eines Längsabschnitts von einer endlosen Bahn abgetrennt worden ist. Das Befestigungselement ist ausgebildet mit mechanisch wirkenden Verschlußelementen, mit einem ersten Abschnitt einer ersten Trägerschicht und mit einem zweiten Abschnitt einer zweiten Trägerschicht, der in Querrichtung neben dem ersten Abschnitt angeordnet und mit diesem verbunden ist, und wobei der erste Abschnitt einen ersten Bereich aufweist, mit dem das Befestigungselement an den Hygieneartikel anfügbar ist, und wobei der zweite Abschnitt einen zweiten Bereich, der in Querrichtung außerhalb des ersten Abschnitts angeordnet ist und in dem die mechanisch wirkenden Verschlußelemente vorgesehen sind, und wobei das Befestigungselement in der Längsrichtung gefaltet ist.

Ein derartiges Befestigungselement ist beispielsweise bekannt aus WO 95/16425 und aus EP 0 669 121 A1.

Des weiteren sind nicht gattungsgemäße Befestigungselemente mit klebenden Verschlußelementen aus US 4,237,890 bekannt, welche jedoch die übrigen vorstehend erwähnten gattungsbildenden Merkmale aufweisen und Z-förmig gefaltet sind. Die Faltung ist derart, dass klebend ausgebildete Bereiche übereinander zu liegen kommen.

Ausgehend von den verschiedenen vorstehend erwähnten bekannten Befestigungselementen, liegt der vorliegenden Erfindung die Aufgabe zugrunde, Befestigungselemente der gattungsgemäßen Art im Hinblick auf die folgenden Gesichtspunkte zu verbessern.

Wenn Befestigungselemente an Hygieneartikel im Herstellungsprozeß der Hygieneartikel angefügt werden, so besteht stets das Problem, die Befestigungselemente in einer Montageposition zu halten, insbesondere wenn der Hygieneartikel nach dem Anfügen in oftmals komplexer Weise gefaltet und verpackt wird. Bei diesen nachfolgenden Handhabungen während der Herstellung der Hygieneartikel tritt oftmals das Problem des Ablösens oder unkontrollierten Auffaltens der Befestigungselemente auf. Hier soll die vorliegende Erfindung Verbesserung schaffen.

Des weiteren soll die Handhabbarkeit der Befestigungselemente und zwar vor und während des Anfügens an einen Hygieneartikel sowie die Anbringbarkeit selbst vereinfacht werden, wobei das zur Verfügung stehende Volumen, welches eine Vielzahl von Befestigungselementen einnehmen, möglichst gering gehalten werden soll.

Diese Aufgabe wird bei einem gattungsgemäßen Befestigungselement erfindungsgemäß dadurch gelöst, dass das Befestigungselement Z-förmig mit in der Längsrichtung verlaufenden Falzlinien gefaltet ist, und dass der die mechanisch wirkenden Verschlußelemente aufweisende zweite Bereich in Querrichtung außerhalb der Z-förmig gefalteten Konfiguration liegt.

Mit der Erfindung wird also vorgeschlagen, eine Z-förmige Faltung derart vorzunehmen und die mechanisch wirkenden Verschlußelemente in einem zweiten Abschnitt der zweiten Trägerschicht so vorzusehen, dass sie außerhalb der Z-förmig gefalteten Konfiguration zu liegen kommen. Dies bringt verschiedene Vorteile bei der Herstellung der Befestigungselemente selbst aber auch bei der Handhabung und beim Anfügen der Befestigungselemente an Hygieneartikel mit sich: Dadurch, dass die mechanisch wirkenden Verschlußelemente, die meist von einer Komponente eines Haken/Schlaufen-Materials gebildet sind, außerhalb der Z-förmig gefalteten Konfiguration zu liegen kommen, besteht nicht die Gefahr, dass ein Auffalten der Befestigungselemente beim bestimmungsgemäßen Einsatz in einem Hygieneartikel dadurch behindert wird, dass sich die Verschlußelemente an der Z-förmigen Faltung verhaken. Ein wesentlicher weiterer Vorteil ist darin zu sehen, dass die mechanisch wirkenden Verschlußelemente nicht mehr in störender Weise in Dickenrichtung auftragen, sondern derart konfigurierbar sind, dass die Dicke des zweiten Abschnitts der Befestigungselemente im Bereich der Verschlußelemente nicht größer ist als im Bereich der Z-förmig gefalteten Konfiguration. Des weiteren eröffnet sich die Möglichkeit, dass die mechanisch wirkenden Verschlußelemente im an einen Hygieneartikel angefügten Zustand der Befestigungselemente mit einer Oberfläche des Hygieneartikels derart haftend zusammenwirken, dass hierdurch ein Ablöseschutz des Befestigungselements erreicht werden kann. Wenn die mechanisch wirkenden Verschlußelemente der Befestigungselemente beispielsweise als hakenbildende Komponente eines Haken/Schlaufen-Materials ausgebildet sind, so können die Haken mit einer textilen Außenbeschichtung einer flüssigkeitsundurchlässigen Außenschicht oder mit einer textilen Komponente einer Innenseite des Hygieneartikels haftend zusammenwirken.

In weiterer Ausbildung der Erfindung erweist es sich als vorteilhaft, wenn eine in der Längsrichtung verlaufende Falzlinie wenigstens abschnittsweise entlang eines Materialübergangs zwischen dem ersten und dem zweiten Abschnitt verläuft. Wenn vorliegend von einem Materialübergang gesprochen wird, so kann hierunter beispielsweise ein überlappender Bereich zwischen der ersten und der zweiten Trägerschicht verstanden werden. Es wäre aber auch denkbar, dass die erste und die zweite Trägerschicht auf Stoß oder in einem verhältnismäßig geringfügigen Abstand zueinander angeordnet und mittels eines dritten, insbesondere streifenförmigen Verbindungselements miteinander verbunden sind. Solchenfalls könnte die besagte Falzlinie entlang eines Materialübergangs zwischen dem ersten oder zweiten Abschnitt und diesem Verbindungselement verlaufen. In jedem Fall vereinfacht sich hierdurch die Falzbarkeit und der Dickenauftrag im Bereich der Z-förmig gefalteten Konfiguration wird möglichst gering gehalten. Vorteilhafterweise verläuft die Falzlinie unmittelbar entlang eines Längsrands des zweiten Abschnitts. Dies erweist sich insbesondere dann als vorteilhaft, wenn der erste und der zweite Abschnitt (schon vor dem Falten) in Querrichtung überlappt sind und im Überlappungsbereich auf an sich beliebige Weise zusammengefügt sind. Als Fügeverfahren kommen in Frage Schweißprozesse oder Klebeverfahren im weitesten Sinne, die eine Verbindung der Materialien mit oder ohne Einschluß eines weiteren Fügematerials bewirken.

Des weiteren wird zur Herstellung der Z-förmigen Faltung vorgeschlagen, dass eine weitere in der Längsrichtung verlaufende Falzlinie unmittelbar entlang eines Längsrands des ersten Bereichs verläuft, mit dem der erste Abschnitt des Befestigungselements an einen Hygieneartikel anfügbar ist. Dieser Bereich ist vorteilhafterweise von einer Kleberbeschichtung gebildet, etwa ein Permanentkleber- oder Haftkleberauftrag, die ihrerseits in Dickenrichtung aufträgt. Es erweist sich somit als vorteilhaft, wenn die weitere Falzlinie wenigstens abschnittsweise entlang eines Längsrands dieses zweiten Bereichs verläuft.

Es erweist sich des weiteren als vorteilhaft, wenn die zweite Trägerschicht ein im wesentlichen unelastisches Material umfaßt und in Querrichtung im wesentlichen nicht dehnbar ausgebildet ist. Beispielsweise könnte die zweite Trägerschicht von einem thermoplastischen Material und/oder einem Vliesmaterial gebildet sein, welches insbesondere und vorzugsweise thermisch gebunden ist, beispielsweise ein aus diskreten Klebepunkten gebildetes Schweiß- oder Prägemuster aufweist. Es kann sich aber auch um eine zumindest in der Querrichtung vorzugsweise im wesentlichen nicht dehnbare Folienschicht handeln.

Die den ersten Abschnitt des Befestigungselements bildende erste Trägerschicht umfaßt vorzugsweise ein zumindest in Querrichtung elastisch dehnbares Material und ist in dieser Querrichtung auch vorzugsweise elastisch dehnbar ausgebildet. Unter elastisch dehnbar wird hierunter ein Bahnmaterial verstanden, welches bei Anwendung von Zugkräften zumindest um das 1,2-Fache seiner Ursprungsabmessung gelängt werden kann und sich nach Wegnahme der Zugkräfte zumindest so weit wieder zusammenzieht, dass zumindest die Hälfte der Längung wieder rückgängig gemacht wird. Es versteht sich, dass Materialien bekannt und für die Verwendung als erste Trägerschicht bevorzugt sind, die ein noch viel weitergehenderes elastisches Retraktionsverhalten zeigen.

Zur Erreichung dieser elastisch dehnbaren Eigenschaften der ersten Trägerschicht können in vorteilhafter Weise elastische Vliesmaterialien oder auch sogenannte "Stretchbond"-Laminate"mit einer oder mehreren Vliesschichten verwendet werden.

In einer weiteren Ausbildung der Erfindung ist die Z-förmig gefaltete Konfiguration auf sich selbst fixiert, was insbesondere durch Klebepunkte oder Schweißpunkte oder durch Vernadelungsprozesse erreicht werden kann. Es erweist sich als zweckmäßig, die Z-förmig gefaltete Konfiguration durch eine Anzahl von 1 bis 10, vorzugsweise 3 bis 5 diskreten Fixierpunkten lösbar zu stabilisieren, wobei die Fixierpunkte mit einer Querschnittsfläche von jeweils weniger als 1 mm² ausgebildet sind.

Nach einem weiteren Erfindungsgedanken von besonderer Bedeutung ist die Z-förmig gefaltete Konfiguration dadurch auf sich selbst fixiert, dass beim Abtrennen der Längsabschnitte in Querrichtung von der endlosen Bahn am hierbei entstehenden Schnittrand Fasern der übereinander gefalteten Lagen in die darunter angeordnete Lage hineingewirkt oder hineingezogen, d.h. aus der einen Lage teilweise ausgezogen und in die andere Lage hineingebracht sind. Der hierbei erzielte Effekt könnte im weitesten Sinne mit einer Vernadelung von Faservliesmaterialien verglichen werden. Beim Abtrennen der Längsabschnitte werden insbesondere durch Ausführung eines "Quetschschnittes" Fasern einer Lage in die in Schnittrichtung darunter angeordnete Lage hineingezogen. Dieser "Quetschschnitt" wird vorzugsweise durch ein nachgiebiges, insbesondere unter Vorspannung stehendes, oder zumindest geringfügig nachgiebig gelagertes Schneidmesser ausgeführt. Dieses Schneidmesser ist vorzugsweise auf einer rotierenden Walze angeordnet und beim Ausführen des Schnitts gegen eine Gegendruckwälze unter entsprechendem Druck anlegbar, über welche die Bahn geführt ist und die ein Widerlager für das Schneidmesser bildet.

Nach einem weiteren Erfindungsgedanken ist in Querrichtung außerhalb des zweiten Bereichs und auf der vom ersten Abschnitt abgewandten Seite des zweiten Bereichs ein zum Ergreifen mit einem Finger des Benutzers dienender Anfaßbereich vorgesehen. In diesem Anfaßbereich sind also keine Verschlußelemente vorgesehen. Er ist vorteilhafterweise von der zweiten Trägerschicht des zweiten Abschnitts selbst gebildet.

Vorteilhafte Dimensionierungen des erfindungsgemäßen Befestigungselements und seiner Bestandteile sind in den Unteransprüchen angegeben, wobei sich die angegebenen Werte auf den Zustand vor dem Falten in Z-förmige Konfiguration beziehen.

Des weiteren erweist es sich bei der Herstellung, aber auch bei der Handhabung des erfindungsgemäßen Befestigungselements als vorteilhaft, wenn der erste Bereich und der zweite Bereich auf derselben Seite der endlosen Bahn vorgesehen sind. Die Z-förmige Faltung ist vorzugsweise derart, dass der erste Bereich und der zweite Bereich auf derselben Sichtseite des gefalteten Befestigungselements vorgesehen sind.

Die vorliegende Erfindung hat ferner zum Gegenstand eine Endlosbahn für die Herstellung von vorstehend beschriebenen erfindungsgemäßen Befestigungselementen, die als Längsabschnitte von dieser Endlosbahn in Querrichtung abgetrennt werden. Diese Endlosbahn ist dadurch gekennzeichnet, dass sie mit der Z-förmig gefalteten Konfiguration in Rollenform aufgewickelt ist.

Die Endlosbahn kann dabei so aufgewickelt sein, dass die Breite der Wicklung durch die Erstreckung der' Befestigungselemente in der Querrichtung gegeben ist oder die Endlosbahn wird kreuzgewickelt, so dass die Breite der Wicklung wesentlich größer als die Quererstreckung der Befestigungselemente ist.

Der Vorteil der erfindungsgemäßen Befestigungselemente und der erfindungsgemäßen Aufwicklung der Endlosbahn ist etwa darin zu sehen, dass der zweite Abschnitt der Befestigungselemente mit den mechanisch ausgebildeten Verschlußelementen im zweiten Bereich nicht in störender Weise dick aufträgt, sondern dass es hingegen möglich ist, die Dicke dieses zweiten Abschnitts im Bereich der Verschlußelemente geringer oder im wesentlichen der Dicke des Befestigungselements im Bereich der Z-förmig gefalteten Konfiguration entsprechend zu wählen.

Nach einem ganz besonders bevorzugten weiteren Erfindungsgedanken erweist es sich als vorteilhaft, wenn bei Befestigungselementen mit einem Kleberauftrag in dem jeweiligen ersten Bereich die Endlosbahn so aufgewickelt ist, dass beim Wickeln der Kleber in dem jeweiligen ersten Bereich auf dem zweiten Abschnitt zu liegen kommt und dort beim Abwickeln leicht lösbar ist. Es wäre sicherlich auch denkbar, dass dieser Kleberauftrag mit einer Release-Beschichtung versehen werden könnte, was aber den Einsatz einer zusätzlichen Schicht bedeuten würde. Insofern erweist es sich als vorteilhaft, wenn der Kleberauftrag und ein entsprechender vorzugsweise in Längsrichtung durchgehender streifenförmiger Bereich des zweiten Abschnitts bzw. dessen Oberfläche so ausgebildet ist, dass ein leichtes Ablösen des Klebers des ersten Bereichs ohne weiteres möglich ist, ohne die Kleberbeschichtung selbst in Mitleidenschaft zu ziehen.

Wenn vorausgehend von einem ersten Abschnitt und einem zweiten Abschnitt des Befestigungselements die Rede war, die miteinander verbunden sind, so wird darauf hingewiesen, dass die z-förmige Faltung in Verbindung mit der Anordnung des die Verschlußelemente tragenden zweiten Bereichs in Querrichtung außerhalb der z-förmig gefalteten Konfiguration auch dann als selbständig erfinderisch angesehen wird, wenn das Befestigungselement anstelle des ersten und zweiten Abschnitts einstückig ausgebildet ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Befestigungselements. In der Zeichnung zeigt:
- Figur 1: eine Schnittansicht einer Ausführungsform eines erfindungsgemäßen Befestigungselements;
- Figur 2: eine Draufsicht auf das Befestigungselement nach Figur 1;
- Figur 3: eine Schnittansicht des Z-förmig gefalteten Befestigungselements nach Figuren 1 und 2;
- Figur 4: eine perspektivische Darstellung einer auf Rollenform gewickelten Endlosbahn;
- Figur 5: eine perspektivische Darstellung einer auf Rollenform gewickelten Endlosbahn, wobei eine Kreuzwicklung vorgesehen ist;
- Figur 6: eine photographische Darstellung des im Auflichtmikroskop betrachteten Schnittrands des Z-förmig gefalteten Befestigungselements und
- Figur 7: eine schematische Darstellung einer Schneidvorrichtung zur Vereinzelung von Z-förmig gefalteten Befestigungselementen.

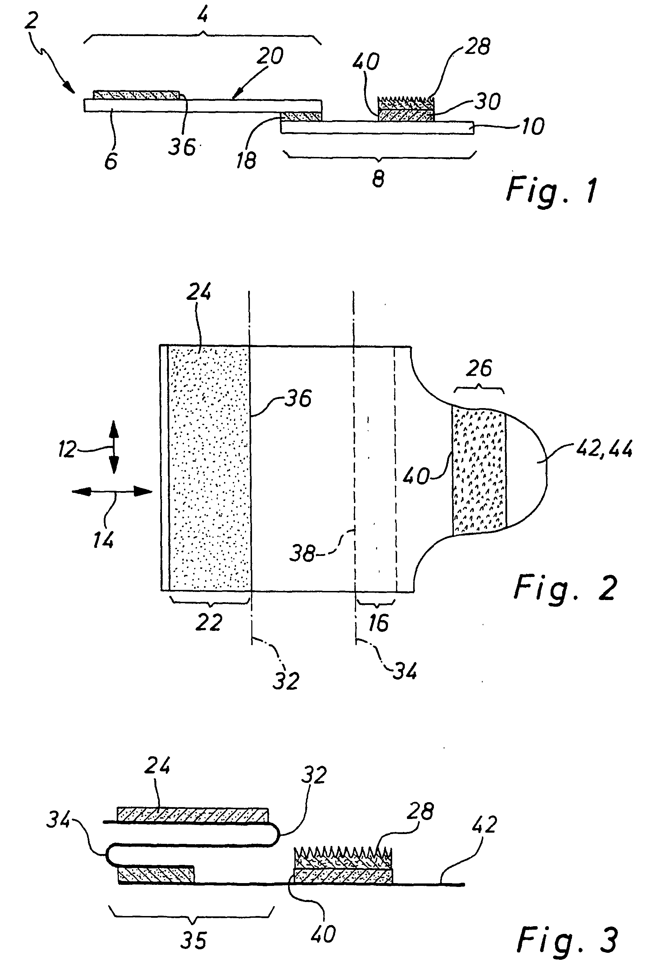

Die Figuren 1 und 2 zeigen ein erfindungsgemäßes Befestigungselement, welches insgesamt mit dem Bezugszeichen 2 bezeichnet ist. Das Befestigungselement 2 umfaßt einen ersten Abschnitt 4 einer ersten Trägerschicht 6 und einen zweiten Abschnitt 8 einer zweiten Trägerschicht 10.

Das Befestigungselement 2 ist als Längsabschnitt von einer endlosen Bahn abgetrennt worden, wobei sich die endlose Bahn in einer ersten Längsrichtung 12 erstreckt. Der zweite Abschnitt 8 ist in einer Querrichtung 14 neben dem ersten Abschnitt 4 angeordnet, wobei im dargestellten Fall der erste Abschnitt 4 und der zweite Abschnitt 8 einander überlappen, so dass ein Überlappungsbereich 16 gebildet ist, an dem die beiden Abschnitte 4, 8 über einen Kleber 18, über Schweißstellen oder in sonstiger Weise unlösbar miteinander verbunden sind.

Auf einer ersten Oberseite 20 des ersten Abschnitts 4 ist ein erster Bereich 22 vorgesehen, der eine Kleberbeschichtung 24 trägt, mit dem das Befestigungselement an einen Hygieneartikel angefügt werden kann.

In einem zweiten Bereich 26 des zweiten Abschnitts 8 sind mechanisch wirkende Verschlußelemente 28, vorzugsweise in Form einer hakenbildenden Komponente eines Haken/Schlaufen-Materials, vorgesehen, insbesondere mittels eines Klebers 30 aufgeklebt. Der zweite Bereich 26 ist vorzugsweise auf derselben Oberseite 20 des Befestigungselements 2 vorgesehen wie der erste Bereich 22.

Des weiteren sind erste und zweite Falzlinien 32, 34 in Figur 2 angedeutet und aus Figur 3 ersichtlich, um welche das Befestigungselement 2 in Längsrichtung 12 Z-förmig gefaltet ist, so dass die in Figur 3 dargestellte Konfiguration 35 gebildet wird. In vorteilhafter Weise verläuft die erste Falzlinie 32 unmittelbar entlang eines Längsrands 36 der Kleberbeschichtung 24 im ersten Bereich 22. Die zweite Falzlinie 34 läuft vorteilhafterweise unmittelbar entlang des Materialübergangs zwischen erstem und zweitem Abschnitt 4 bzw. 8, also entlang eines Rands 38 des Überlappungsbereichs 16.

Wie aus Figur 3 ersichtlich ist der zweite Bereich 26 des zweiten Abschnitts 8 in Querrichtung 14 derart weit vom Überlappungsbereich 16 oder von einem sonstigen Materialübergangsbereich zwischen erstem Abschnitt 4 und zweitem Abschnitt 8 beabstandet, dass er sich in Querrichtung 14 außerhalb der Z-förmig gefalteten Konfiguration 35 des Befestigungselements 2 befindet, was sich in mehrfacher Hinsicht als vorteilhaft erweisen kann. Im dargestellten Fall reicht ein dem ersten Abschnitt 4 zugewandter Längsrand 40 des zweiten Bereichs 26 und damit der mechanisch wirkenden Verschlußelemente 28 in Querrichtung 14 nahezu an die erste Falzlinie 36 bei Z-förmig gefalteter Konfiguration 25 heran. Es wäre aber auch denkbar, dass der zweite Bereich 26 so bezüglich des zweiten Abschnitts 8 positioniert ist, dass die mechanisch wirkenden Verschlußelemente 28 bzw. deren Längsrand 40 einen Abstand von einigen Millimetern von der Z-förmig gefalteten Konfiguration 35 des Befestigungselements aufweisen.

Wenn das Befestigungselement mit seinem Haftkleberauftrag 24 im ersten Bereich 22 auf einen Hygieneartikel aufgebracht ist, so ist - wie bereits erwähnt - der zweite Bereich 26 mit den mechanisch wirkenden Verschlußelementen 28 außerhalb der Z-förmig gefalteten Konfiguration 35 angeordnet und kann somit zum Festlegen des Befestigungselements bzw. des zweiten Abschnitts 8 auf einer textilen Oberfläche eines Hygieneartikels dienen. Dieses Festlegen des zweiten Abschnitts 8 soll aber lediglich der Fixierung des Befestigungselements während der Herstellung und Verpackung bis zum Anlegen des Hygieneartikels an einen Benutzer dienen, zu welchem Zeitpunkt spätestens diese Verbindung gelöst wird. Ein Benutzer greift dann mit seinen Fingern zwischen die Oberseite des Hygieneartikels und einen freien Endbereich 42 des zweiten Abschnitts 8, der dann als Anfaßbereich 44 dient.

Es sei noch erwähnt, dass der erste Abschnitt 4 in Querrichtung 14 elastisch dehnbar ausgebildet ist und dass der zweite Abschnitt 8 in Querrichtung 14 im wesentlichen nicht dehnbar ausgebildet ist.

Eine Kleberbeschichtung des ersten Bereichs 24 sowie mechanisch wirkende Verschlußelemente im zweiten Bereich 26 können in Längsrichtung 12 durchgehend und endlos auf eine dementsprechend endlose Bahn der ersten Trägerschicht 6 und der zweiten Trägerschicht 8 aufgebracht werden. In entsprechender Weise werden die erste und die zweite Trägerschicht 6, 8 durch einen endlosen Kleberstreifen 18 oder in sonstiger Weise in Längsrichtung 12 kontinuierlich miteinander verbunden. Eine Simultanfertigung zweier spiegelbildlich jedoch in Längsrichtung um n/2 versetzt, wie dies beispielsweise aus EP 0 669 121 A1 bekannt ist, ist ebenfalls denkbar und vorteilhaft.

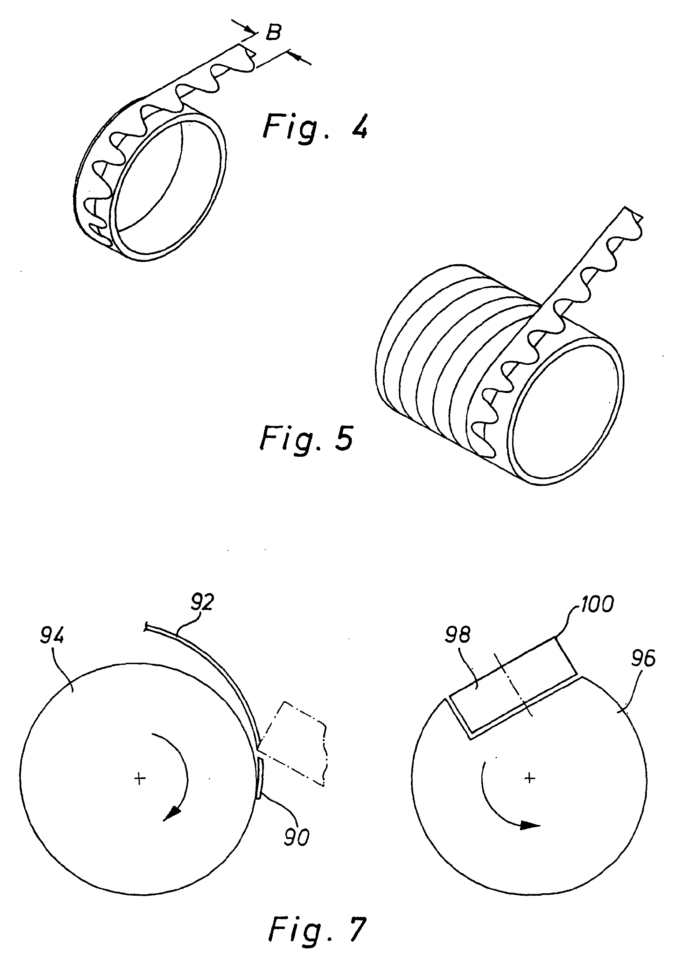

Schließlich zeigen die Figuren 4 und 5 jeweils eine Endlosbahn mit noch nicht als Längsabschnitte in Querrichtung 14 abgetrennten Befestigungselementen in aufgewickelter Form, wobei Figur 5 eine Kreuzwicklung zeigt, bei der die Breite der Wicklung wesentlich größer als die jeweilige Breite oder Quererstreckung der einzelnen Befestigungselemente 2 ist. Hierdurch wird aber insgesamt eine größere Wicklungsstabilität erreicht und es können auf einer einzigen Rolle größere Mengen von Befestigungselementen aufgewickelt und für einen Produktionsprozeß vorgehalten werden.

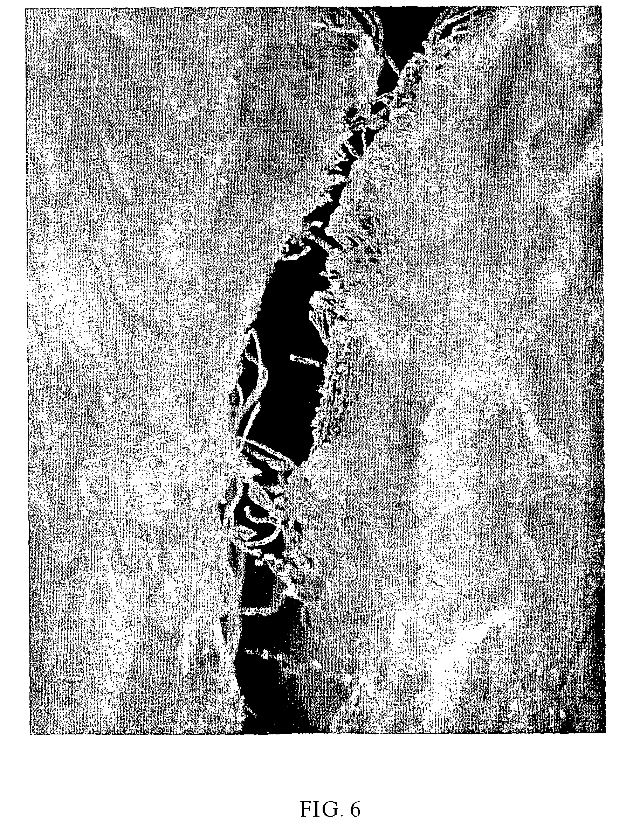

Zur Fixierung der Befestigungselemente 2 in Z-förmig gefalteter Konfiguration 35 ist es denkbar, diskrete Fixierpunkte, etwa Schweißpunkte vorzusehen, welche die Z-förmig übereinander gefalteten Lagen des Befestigungselements 2 lösbar miteinander verbinden. Es hat sich aber gezeigt, dass eine den Anforderungen genügende Fixierung der Z-förmig gefalteten Konfiguration 35 dadurch erreicht werden kann, dass beim Abtrennen der die jeweiligen Befestigungselemente bildenden Längsabschnitte von einer Endlosbahn Fasern der übereinander angeordneten, Z-förmig gefalteten Lagen in die jeweils darunter liegende Lage hinein gezogen werden, so dass eine Art Vernadelungseffekt entsteht, der die Z-förmige Konfiguration 35 lösbar auf sich selbst fixiert. Dies zeigt Figur 6.

Figur 7 zeigt in schematischer Andeutung eine Schneidvorrichtung zum Abtrennen von Längsabschnitten 90 von einer Endlosbahn 92, welche zur Bildung der Befestigungselemente schon die Z-förmig gefaltete Konfiguration aufweist. Die Schneidvorrichtung umfasst eine Ambosswalze 94, über welche die Endlosbahn 92 geführt ist und eine Messerwalze 96 mit einem unter Vorspannung stehenden und schwimmend gelagerten oder in sich nachgiebigen Schneidmesser 98 mit einer Schneidkante 100. Zur Ausführung des Schnitts ist die Scheidwalze 96 derart gegenüber der Ambosswalze 94 positioniert, dass die Schnittkante 100 des Schneidmessers 98 die Oberfläche der Ambosswalze 94 berührt und geringfügig ausweichen kann. Die Endlosbahn 92 wird also über einen zwar verhältnismäßig kurzen Umfangsbereich von der Schneidkante 100 des Schneidmessers 98 beaufschlagt. Hierdurch werden im Unterschied zu einem bloßen Abscheren Fasern der übereinander angeordneten Lagen bei der Ausführung dieses "Quetschschnittes" aus der einen Lage ausgelenkt und in die darunterliegende Lage hineingezogen, hineingequetscht oder hineingewirkt, so dass im abgetrennten Längsabschnitt 90 die Z-förmig übereinander gefalteten Lagen zumindest geringfügig miteinander verbunden sind und so ein unbeabsichtigtes Auffalten vermieden wird.

## Patentansprüche

1. Befestigungselement (2) für Hygieneartikel, insbesondere für Windeln oder für Inkontinenzartikel, zum lösbaren Schließen des Hygieneartikels im an einen Benutzer angelegten Zustand, wobei das Befestigungselement (2) eine erste Längsrichtung (12) und eine zweite Querrichtung (14) aufweist und in Form eines Längsabschnitts von einer endlosen Bahn abgetrennt worden ist, mit mechanisch wirkenden Verschlußelementen (28), mit einem ersten Abschnitt (4) einer ersten Trägerschicht (6) und mit einem zweiten Abschnitt (8) einer zweiten Trägerschicht (10), der in Querrichtung (14) neben dem ersten Abschnitt (4) angeordnet und mit diesem verbunden ist, und wobei der erste Abschnitt (4) einen ersten Bereich (22) aufweist, mit dem das Befestigungselement an den Hygieneartikel anfügbar ist, und wobei der zweite Abschnitt (8) einen zweiten Bereich (26) aufweist, der in Querrichtung (14) außerhalb des ersten Abschnitts (4) angeordnet ist und in dem die mechanisch wirkenden Verschlußelemente (28) vorgesehen sind, wobei das Befestigungselement in der Längsrichtung (12) gefaltet ist, **dadurch gekennzeichnet, dass** das Befestigungselement z-förmig mit in der Längsrichtung (12) verlaufenden Falzlinien (32, 34) gefaltet ist, und dass der die mechanisch wirkenden Verschlußelemente (28) aufweisende zweite Bereich (26) in Querrichtung (14) außerhalb der z-förmig gefalteten Konfiguration (35) liegt.

2. Befestigungselement nach Anspruch 1, **dadurch gekennzeichnet, dass** eine in der Längsrichtung (12) verlaufende Falzlinie (34) wenigstens abschnittsweise entlang eines Materialübergangs zwischen erstem und zweitem Abschnitt (4, 8) verläuft.

3. Befestigungselement nach Anspruch 2, **dadurch gekennzeichnet, dass** die Falzlinie (34) unmittelbar entlang eines Längsrands (38) des zweiten Abschnitts (8) verläuft.

4. Befestigungselement nach Anspruch 1, 2 oder 3, **dadurch gekennzeichnet, dass** der erste und der zweite Abschnitt (4, 8) in Querrichtung (14) überlappt sind und im Überlappungsbereich (16) zusammengefügt sind.

5. Befestigungselement nach Anspruch 4, **dadurch gekennzeichnet, dass** eine in der Längsrichtung (12) verlaufende Falzlinie (32) unmittelbar entlang eines Längsrands (36) des ersten Bereichs (22) verläuft.

6. Befestigungselement nach Anspruch 4 oder 5, **dadurch gekennzeichnet, dass** die Falzlinie (32) unmittelbar entlang eines Längsrands (36) einer Kleberbeschichtung (24) im ersten Bereich (22) verläuft.

7. Befestigungselement nach einem oder mehreren der vorstehenden Ansprüche, **dadurch gekennzeichnet, dass** die zweite Trägerschicht (10) ein im wesentlichen unelastisches Material umfasst und in Querrichtung (14) im wesentlichen nicht dehnbar ausgebildet ist.

8. Befestigungselement nach einem oder mehreren der vorstehenden Ansprüche, **dadurch gekennzeichnet, dass** die erste Trägerschicht (6) ein zumindest in Querrichtung (14) elastisch dehnbares Material umfasst und in dieser Querrichtung (14) elastisch dehnbar ausgebildet ist.

9. Befestigungselement nach einem oder mehreren der vorstehenden Ansprüche, **dadurch gekennzeichnet, dass** der erste Bereich (22) klebend ausgebildet ist.

10. Befestigungselement nach einem oder mehreren der vorstehenden Ansprüche, **dadurch gekennzeichnet, dass** die z-förmig gefaltete Konfiguration (35) auf sich selbst fixiert ist.

11. Befestigungselement nach Anspruch 10, **dadurch gekennzeichnet, dass** die z-förmig gefaltete Konfiguration (35) auf sich selbst fixiert ist durch eine Anzahl von 1-10, vorzugsweise 3-5 diskreten Fixierpunkten mit einer Querschnittsfläche von jeweils weniger als 1 mm².

12. Befestigungselement nach Anspruch 10 oder 11, **dadurch gekennzeichnet, dass** die z-förmig gefaltete Konfiguration (35) dadurch auf sich selbst fixiert ist, dass beim Abtrennen der Längsabschnitte (90) in Querrichtung (14) von der endlosen Bahn am Schnittrand Fasern der übereinander gefalteten Lagen in die darunter angeordnete Lage hineingewirkt oder hineingequetscht sind.

13. Befestigungselement nach Anspruch 11 oder 12, **dadurch gekennzeichnet, dass** in Querrichtung (14) außerhalb des zweiten Bereichs (26) und auf der vom ersten Abschnitt (4) abgewandten Seite des zweiten Bereichs (26) ein zum Ergreifen mit einem Finger des Benutzers dienender Anfassbereich (44) vorgesehen ist.

14. Befestigungselement nach Anspruch 13, **dadurch gekennzeichnet, dass** der Anfassbereich (44) von der zweiten Trägerschicht (10) des zweiten Abschnitts (8) selbst gebildet ist.

15. Befestigungselement nach Anspruch 14, **dadurch gekennzeichnet, dass** seine Erstreckung in der Längsrichtung 2-10 cm, insbesondere 5-7 cm, beträgt.

16. Befestigungselement nach Anspruch 15, **dadurch gekennzeichnet, dass** die Erstreckung des ersten Abschnitts (4) in der Querrichtung (14) in einem elastisch ausgebildeten Bereich zwischen dem Längsrand (6) des ersten Bereichs (22) und dem Materialübergang zum zweiten Abschnitt (8) 0,5 - 4,5 cm, insbesondere 2-3 cm, beträgt.

17. Befestigungselement nach Anspruch 16, **dadurch gekennzeichnet, dass** die Erstreckung des die mechanisch wirkenden Verschlußelemente (28) aufweisenden zweiten Bereichs (26) in der Querrichtung (14) 0, 5-2, 1 cm, insbesondere 1-1,5 cm, beträgt.

18. Befestigungselement nach Anspruch 17, **dadurch gekennzeichnet, dass** die Erstreckung des Anfassbereichs (44) in der Querrichtung (14) 0,5 - 2,0 cm, insbesondere 0,8-1,5 cm beträgt.

19. Befestigungselement nach Anspruch 18, **dadurch gekennzeichnet, dass** die Erstreckung des insbesondere klebend ausgebildeten ersten Bereichs (22) des ersten Abschnitts (4) in Querrichtung (14) im wesentlichen der Breite der z-förmig gefalteten Konfiguration (35) entspricht.

20. Befestigselement nach Anspruch 19, **dadurch gekennzeichnet, dass** der erste Bereich (22) und der zweite Bereich (26) auf derselben Seite (20) der endlosen Bahn vorgesehen sind.

21. Befestigungselement nach Anspruch 20, **dadurch gekennzeichnet, dass** die z-förmige Faltung derart ist, dass der erste Bereich (22) und der zweite Bereich (26) auf derselben Sichtseite des gefalteten Befestigungselements vorgesehen sind.

22. Befestigungselement nach Anspruch 21, **dadurch gekennzeichnet, dass** die Dicke (senkrecht zur Bahnebene) der z-förmig gefalteten Konfiguration (35) größer ist als die Dicke des zweiten Abschnitts (8) im Bereich des zweiten die mechanisch wirkenden Verschlußelemente (28) aufweisenden Bereichs (26).

23. Endlosbahn für die Herstellung von Befestigungselementen für Hygieneartikel, insbesondere für Windeln oder für Inkontinenzartikel, zum lösbaren Schließen des Hygieneartikels im an einen Benutzer angelegten Zustand, wobei das Befestigungselement (2) eine erste Längsrichtung (12) und eine zweite Querrichtung (14) aufweist und in Form eines Längsabschnitts von einer endlosen Bahn abgetrennt worden ist, mit mechanisch wirkenden Verschlußelementen (28), mit einem ersten Abschnitt (4) einer ersten Trägerschicht (6) und mit einem zweiten Abschnitt (8) einer zweiten Trägerschicht (10), der in Querrichtung (14) neben dem ersten Abschnitt (4) angeordnet und mit diesem verbunden ist, und wobei der erste Abschnitt (4) einen ersten Bereich (22) aufweist, mit dem das Befestigungselement an den Hygieneartikel anfügbar ist, und wobei der zweite Abschnitt (8) einen zweiten Bereich (26) aufweist, der in Querrichtung (14) außerhalb des ersten Abschnitts (4) angeordnet ist und in dem die mechanisch wirkenden Verschlußelemente (28) vorgesehen sind, wobei das Befestigungselement in der Längsrichtung (12) gefaltet ist und wobei das Befestigungselement z-förmig mit in der Längsrichtung (12) verlaufenden Falzlinien (32, 34) gefaltet ist, und dass der die mechanisch wirkenden Verschlußelemente (28) aufweisende zweite Bereich (26) in Querrichtung (14) außerhalb der z-förmig gefalteten Konfiguration (35) liegt, wobei die Befestigungselemente als Längsabschnitte von der Endlosbahn in Querrichtung abgetrennt werden,
**dadurch gekennzeichnet,**
**dass** die Endlosbahn mit der z-förmig gefalteten Konfiguration (35) in Rollenform aufgewickelt ist.

24. Endlosbahn nach Anspruch 23, **dadurch gekennzeichnet, dass** die Endlosbahn so aufgewickelt ist, dass die Breite (B) der Wicklung durch die Erstreckung der Befestigungselemente (2) in Querrichtung (14) gegeben ist.

25. Endlosbahn nach Anspruch 23, **dadurch gekennzeichnet, dass** die Endlosbahn kreuzgewickelt ist, so dass die Breite (B') der Wicklung wesentlich größer als die Erstreckung der Befestigungselemente (2) in Querrichtung (14) ist.

26. Endlosbahn nach den Ansprüchen 23, 24 oder 25, **dadurch gekennzeichnet, dass** zumindest in dem jeweiligen ersten Bereich (22) des ersten Abschnitts (8) ein Kleberaufrag (24) vorgesehen ist und dass beim Wickeln der Endlosbahn der Kleberauftrag (24) in dem jeweiligen ersten Bereich (22) auf dem zweiten Abschnitt (8) zu liegen kommt und dort beim Abwickeln leicht ablösbar ist.

## Claims

1. Fastening element (2) for hygiene articles, in particular for nappies or for incontinence articles, for the releasable closure of the hygiene article when applied to a user, the fastening element (2) having a first longitudinal direction (12) and a second transverse direction (14) and having been separated from a continuous web in the form of a longitudinal portion, with mechanically acting closures (28), with a first portion (4) of a first supporting layer (6), with a first portion (4) of a first supporting layer (6) and with a second portion (8) of a second supporting layer (10), which is disposed in a transverse direction (14) next to the first portion (4) and connected thereto, and the first portion (4) having a first region (22) with which the fastening element can be attached to the hygiene article, and with the second portion (8) having a second region (26), which is disposed in the transverse direction (14) outside the first portion (4) and in which the mechanically acting closures (28) are provided, the fastening element being folded in the longitudinal direction (12), **characterised in that** the fastening element is folding in a Z-shape with the folding lines (32, 34) running in longitudinal direction (12), and the second region (26) comprising the mechanically acting closures (28) lying in the transverse direction (14) outside the Z-shape folded configuration (35).

2. Fastening element according to claim 1, **characterised in that** a fold line (34) running in the longitudinal direction (12) runs at least in portions along a material transition between the first and second portion (4, 8).

3. Fastening element according to claim 2, **characterised in that** the fold line (34) runs immediately along a longitudinal edge (38) of the second portion (8).

4. Fastening element according to claim 1, claim 2 or claim 3, **characterised in that** the first and the second portion (4, 8) are overlapped in the transverse direction (14) and are joined in the overlapping region (16).

5. Fastening element according to claim 4, **characterised in that** a fold line (32) running in the longitudinal direction (12) runs immediately along a longitudinal edge (36) of the first region (22).

6. Fastening element according to claim 4 or claim 5, **characterised in that** the fold line (32) runs immediately along one longitudinal edge (36) of an adhesive coating (24) in the first region (22).

7. Fastening element according to one or more of the preceding claims, **characterised in that** the second supporting layer (10) comprises a substantially unresilient material and is configured to be substantially not extensible in the transverse direction (14).

8. Fastening element according to one or more of the preceding claims, **characterised in that** the first supporting layer (6) comprises a resiliently extensible material at least in the transverse direction (14) and is configured to be substantially extensible in this transverse direction (14).

9. Fastening element according to one or more of the preceding claims, **characterised in that** the first region (22) is configured to be adhesive.

10. Fastening element according to one or more of the preceding claims, **characterised in that** the Z-shape folded configuration (35) is secured to itself.

11. Fastening element according to claim 10, **characterised in that** the Z-shape folded configuration (35) is secured to itself by 1 to 10, preferably 3 to 5 discrete fixing points, each with a cross sectional area of less than 1 mm².

12. Fastening element according to claim 10 or claim 11, **characterised in that** the Z-shape folded configuration (35) is secured to itself such in a manner that, when the longitudinal portions (90) are separated in the transverse direction (14) from the continuous web, fibres from the cut edge of the layers folded over on each other are worked into or squeezed into the layer disposed thereunder.

13. Fastening element according to claim 11 or claim 12, **characterised in that** a pull-tab (44) is provided in the transverse direction (14) outside the second region (26) and on the side of the second region (26) facing away from the first portion for the user to grasp with a finger.

14. Fastening element according to claim 13, **characterised in that** the pull-tab (44) is configured by the second supporting layer (10) of the second portion (8) itself.

15. Fastening element according to claim 14, **characterised in that** the extension of the fastening element in the longitudinal direction is about 2 to 10 cm, in particular 5 to 7 cm.

16. Fastening element according to claim 15, **characterised in that** the extension of the first portion (4) in the transverse direction (14) in a resiliently configured region between the longitudinal edge (6) of the first region (22) and the material transition with the second portion (8) is 0.5 to 4.5 cm, in particular 2 to 3 cm.

17. Fastening element according to claim 16, **characterised in that** the extension of the second region (26) having the mechanically acting closures (28) is 0.5 to 2.1 cm, in particular 1 to 1.5 cm, in the transverse direction (14).

18. Fastening element according to claim 17, **characterised in that** the extension of the pull-tab (44) in the transverse direction (14) measures 0.5 to 2.0 cm, in particular 0.8 to 1.5 mm.

19. Fastening element according to claim 18, **characterised in that** the extension of the particularly adhesively configured first region (22) of the first portion (4) in the transverse direction (14) corresponds substantially to the width of the Z-shape folded configuration (35).

20. Fastening element according to claim 19, **characterised in that** the first region (22) and the second region (26) are provided on the same side (20) of the continuous web.

21. Fastening element according to claim 20, **characterised in that** the Z-shaped fold is such that the first region (22) and the second region (26) are provided on the same visible side of the folded fastening element.

22. Fastening element according to claim 21, **characterised in that** the thickness (vertical to the surface of the web) of the Z-shape folded configuration (35) is greater than a thickness of the second portion (8) in the region of the second region (26) having the mechanically acting closures (28).

23. Continuous web for the production of fastening elements, in particular for nappies or for incontinence articles for the releasable closure of the hygiene article when applied to a user, the fastening element (2) having a first longitudinal direction (12) and a second transverse direction (14) and having been separated from a continuous web in the form of a longitudinal portion, with mechanically acting closures (28), with a first portion (4) of a first supporting layer (6), with a first portion (4) of a first supporting layer (6) and with a second portion (8) of a second supporting layer (10), which is disposed in a transverse direction (14) next to the first portion (4) and connected thereto, and the first portion (4) having a first region (22), with which the fastening element can be attached to the hygiene article, and the second portion (8) having a second region (26), which is disposed in the transverse direction (14) outside the first portion (4) and in which the mechanically acting closures (28) are provided, the fastening element being folded in the longitudinal direction (12), and the fastening element being folded in a Z-shape with the folding lines (32, 34) running in the longitudinal direction (12), and the second region (26) comprising the mechanically acting closures (28) lying in the transverse direction (14) outside the Z-shape folded configuration (35), wherein the fastening elements are separated as longitudinal portions in the transverse direction from the continuous web, **characterised in that** the continuous web the Z-shape folded configuration (35).is wound in the form of rolls

24. Continuous web according to claim 23, **characterised in that** the continuous web is wound in such a way that the width (B') of the coil is determined by the extension of the fastening elements (2) in the transverse direction (14).

25. Continuous web according to claim 23, **characterised in that** the continuous web is cross-wound so that the width (B') of the coil is considerably greater than the extension of the fastening elements (2) in the transverse direction (14).

26. Continuous web according to claim 23, claim 24 or claim 25, **characterised in that** an adhesive application (24) is provided in the particular first region (22) and during the winding of the continuous web at least in the adhesive application (24) comes to lie on the second portion (8) in the respective first region (22) and can easily be detached therefrom when the web is unwound.

## Revendications

1. Élément de fixation (2) pour articles d'hygiène, en particulier pour des couches ou pour des articles pour incontinence, destiné à la fermeture détachable de l'article d'hygiène lorsque celui-ci est placé sur un utilisateur, l'élément de fixation (2) présentant un premier sens longitudinal (12) et un deuxième sens transversal (14) et ayant été séparé d'une bande sans fin sous la forme d'un segment longitudinal, avec des éléments de fermeture à action mécanique (28), avec un premier segment (4) d'une première couche porteuse (6) et avec un deuxième segment (8) d'une deuxième couche porteuse (10) qui est agencé dans le sens transversal (14) à côté du premier segment (4) et relié à celui-ci, et le premier segment (4) présentant une première zone (22) avec laquelle l'élément de fixation peut être joint à l'article d'hygiène, et le deuxième segment (8) présentant une deuxième zone (26) qui est agencée dans le sens transversal (14) en dehors du premier segment (4) et dans laquelle sont prévus les éléments de fermeture à action mécanique (28), l'élément de fixation étant plié dans le sens longitudinal (12), **caractérisé en ce que** l'élément de fixation est plié en forme de z avec des lignes de pliage (32, 34) s'étendant dans le sens longitudinal (12) et **en ce que** la deuxième zone (26) présentant les éléments de fermeture à action mécanique (28) est située dans le sens transversal (14) en dehors de la configuration pliée en forme de z (35).

2. Élément de fixation selon la revendication 1, **caractérisé en ce qu'**une ligne de pliage (34) s'étendant dans le sens longitudinal (12) s'étend au moins par segments le long d'un passage de matériau entre le premier et le deuxième segment (4, 8).

3. Élément de fixation selon la revendication 2, **caractérisé en ce que** la ligne de pliage (34) s'étend directement le long d'un bord longitudinal (38) du deuxième segment (8).

4. Élément de fixation selon la revendication 1, 2 ou 3, **caractérisé en ce que** le premier et le deuxième segments (4, 8) se chevauchent dans le sens transversal (14) et sont assemblés dans la zone de chevauchement (16).

5. Élément de fixation selon la revendication 4, **caractérisé en ce qu'**une ligne de pliage (32) s'étendant dans le sens longitudinal (12) s'étend directement le long d'un bord longitudinal (36) de la première zone (22).

6. Élément de fixation selon la revendication 4 ou 5, **caractérisé en ce que** la ligne de pliage (32) s'étend directement le long d'un bord longitudinal (36) d'un revêtement adhésif (24) dans la première zone (22).

7. Élément de fixation selon l'une ou plusieurs des revendications précédentes, **caractérisé en ce que** la deuxième couche porteuse (10) comprend un matériau essentiellement non élastique et est conçue essentiellement non extensible dans le sens transversal (14).

8. Élément de fixation selon l'une ou plusieurs des revendications précédentes, **caractérisé en ce que** la première couche porteuse (6) comprend un matériau extensible élastiquement au moins dans le sens transversal (14) et est conçue extensible élastiquement dans ce sens transversal (14).

9. Élément de fixation selon l'une ou plusieurs des revendications précédentes, **caractérisé en ce que** la première zone (22) est réalisée adhésive.

10. Élément de fixation selon l'une ou plusieurs des revendications précédentes, **caractérisé en ce que** la configuration pliée en forme de z (35) est fixée sur elle-même.

11. Élément de fixation selon la revendication 10, **caractérisé en ce que** la configuration pliée en forme de z (35) est fixée sur elle-même par un nombre de points de fixation allant de 1 à 10, de préférence de 3 à 5, ayant chacun une aire de section transversale inférieure à 1 mm².

12. Élément de fixation selon la revendication 10 ou 11, **caractérisé en ce que** la configuration pliée en forme de z (35) est fixée sur elle-même **en ce que**, lors de la séparation des segments longitudinaux (90) d'avec la bande sans fin dans le sens transversal (14) au bord de coupe, des fibres des couches pliées les unes sur les autres sont enchevêtrées ou écrasées dans la couche située au-dessous.

13. Élément de fixation selon la revendication 11 ou 12, **caractérisé en ce que**, dans le sens transversal (14) en dehors de la deuxième zone (26) et du côté de la deuxième zone (26) opposé au premier segment (4), il est prévu une zone de prise (44) destinée à être saisie par un doigt de l'utilisateur.

14. Élément de fixation selon la revendication 13, **caractérisé en ce que** la zone de prise (44) est elle-même formée par la deuxième couche porteuse (10) du deuxième segment (8).

15. Élément de fixation selon la revendication 14, **caractérisé en ce que** son étendue dans le sens longitudinal est comprise entre 2 et 10 cm, en particulier entre 5 et 7 cm.

16. Élément de fixation selon la revendication 15, **caractérisé en ce que** l'étendue du premier segment (4) dans le sens transversal (14) dans une zone conçue élastique entre le bord longitudinal (6) de la première zone (22) et le passage de matériau au deuxième segment (8) est comprise entre 0,5 et 4,5 cm, en particulier entre 2 et 3 cm.

17. Élément de fixation selon la revendication 16, **caractérisé en ce que** l'étendue de la deuxième zone (26) présentant les éléments de fermeture à action mécanique (28) dans le sens transversal (14) est comprise entre 0,5 et 2,1 cm, en particulier entre 1 et 1,5 cm.

18. Élément de fixation selon la revendication 17, **caractérisé en ce que** l'étendue de la zone de prise (44) dans le sens transversal (14) est comprise entre 0,5 et 2 cm, en particulier entre 0,8 et 1,5 cm.

19. Élément de fixation selon la revendication 18, **caractérisé en ce que** l'étendue de la première zone (22) conçue en particulier adhésive du premier segment (4) dans le sens transversal (14) correspond essentiellement à la largeur de la configuration pliée en forme de z (35).

20. Élément de fixation selon la revendication 19, **caractérisé en ce que** la première zone (22) et la deuxième zone (26) sont prévues du même côté (20) de la bande sans fin.

21. Élément de fixation selon la revendication 20, **caractérisé en ce que** le pliage en forme de z est tel que la première zone (22) et la deuxième zone (26) sont prévues du même côté de la vue de l'élément de fixation plié.

22. Élément de fixation selon la revendication 21, **caractérisé en ce que** l'épaisseur (perpendiculairement au plan de la bande) de la configuration pliée en forme de z (35) est supérieure à l'épaisseur du deuxième segment (8) dans la zone de la deuxième zone (26) présentant les éléments de fermeture à action mécanique (28).

23. Bande sans fin pour la fabrication d'éléments de fixation pour articles d'hygiène, en particulier pour des couches ou pour des articles pour incontinence, destinée à la fermeture détachable de l'article d'hygiène lorsque celui-ci est placé sur un utilisateur, l'élément de fixation (2) présentant un premier sens longitudinal (12) et un deuxième sens transversal (14) et ayant été séparé d'une bande sans fin sous la forme d'un segment longitudinal, avec des éléments de fermeture à action mécanique (28), avec un premier segment (4) d'une première couche porteuse (6) et avec un deuxième segment (8) d'une deuxième couche porteuse (10) qui est agencé dans le sens transversal (14) à côté du premier segment (4) et est relié à celui-ci et le premier segment (4) présentant une première zone (22) par laquelle l'élément de fixation peut être joint à l'article d'hygiène et le deuxième segment (8) présentant une deuxième zone (26) qui est agencée dans le sens transversal (14) en dehors du premier segment (4) et dans laquelle les éléments de fermeture à action mécanique (28) sont prévus, l'élément de fixation étant plié dans le sens longitudinal (12) et l'élément de fixation étant plié en forme de z avec des lignes de pliage (32, 34) s'étendant dans le sens longitudinal (12) et la deuxième zone (26) présentant les éléments de fermeture à action mécanique (28) étant située dans le sens transversal (14) en dehors de la configuration pliée en forme de z (35), les éléments de fixation en tant que segments longitudinaux étant séparés de la bande sans fin dans le sens transversal, **caractérisée en ce que** la bande sans fin est enroulée en forme de rouleau avec la configuration pliée en forme de z (35).

24. Bande sans fin selon la revendication 23, **caractérisée en ce que** la bande sans fin est enroulée de telle manière que la largeur (B) de l'enroulement est donnée par l'étendue des éléments de fixation (2) dans le sens transversal (14).

25. Bande sans fin selon la revendication 23, **caractérisée en ce que** la bande sans fin est enroulée en croix de telle manière que la largeur (B') de l'enroulement est sensiblement plus grande que l'étendue des éléments de fixation (2) dans le sens transversal (14).

26. Bande sans fin selon les revendications 23, 24 ou 25, **caractérisée en ce qu'**il est prévu une couche adhésive (24) au moins dans la première zone (22) du premier segment (8) et que, lors de l'enroulement de la bande sans fin, la couche adhésive (24) vient en contact sur le deuxième segment (8) dans la première zone (22) et est facilement détachable à cet endroit lors du déroulement.
